# EUROPÄISCHE PATENTSCHRIFT

(11) **EP 1 171 182 B1**
(45) Veröffentlichungstag und Bekanntmachung des Hinweises auf die Patenterteilung: **19.11.2008**
(21) Anmeldenummer: 00912848.9
(22) Anmeldetag: 03.04.2000
(51) Int. Cl.: A61M 16/00

(54) **BEATMUNGSGERÄT**
BREATHING APPARATUS
APPAREIL RESPIRATOIRE

(30) Priorität: 07.04.1999 CH 65299
(43) Veröffentlichungstag der Anmeldung: 16.01.2002
(73) Patentinhaber: eVent Medical Limited, Galway (IE)
(72) Erfinder: FRIBERG, Harri, CH-9470 Buchs (CH); DÄSCHER, Jakob, CH-9470 Buchs (CH)
(74) Vertreter: Rosenich, Paul
(86) Internationale Anmeldenummer: PCT/IB2000/000407
(87) Internationale Veröffentlichungsnummer: WO 2000/059566

(56) Entgegenhaltungen:
- EP-A- 0 266 964
- EP-A- 0 643 978
- EP-A- 0 903 160
- DE-A- 3 336 578
- US-A- 4 054 133

## Beschreibung

Die Erfindung betrifft ein Beatmungsgerät nach dem Oberbegriff des Anspruches 1.

### Stand der Technik

Beatmungsgeräte dienen der Beatmung von Patienten mit einer Atemschwäche, mit einem Ausfall der Lungenfunktion oder als Gasmischgeräte zur Konditionierung der Atemluft eines Patienten. Demzufolge verfügen sie über Atemgasanschlüsse, Ventile, Steuerungen für die Ventile und Druckgasanschlüsse zur Erzeugung eines Gasdruckes für das Einpressen von Luft in Beatmungsschläuche bzw. in die Lungen eines Patienten.

Im Betrieb sind die Druckgasanschlüsse häufig mit einem Druckluftnetz verbunden, wobei die Druckluft im Gerät eine druckluftbetriebene Pumpe betätigt, die die Atemgase fördert. Es können aber auch die Atemgase über die Atemgasanschlüsse selbst unter Druck eingespeist werden, so dass der erforderlichen Druck für die Beatmung von den Atemgasen selbst geliefert wird. Es sind auch Beatmungsgeräte bekannt, die auf Kompressoren aufgesetzt werden können, welche letztere im Betrieb die erforderlichen Atemgasdrücke erzeugen und in das Beatmungsgerät einspeisen.

Herkömmliche Beatmungsgeräte verfügen demzufolge über ein Gehäuse, in dem Gasvorratsbehälter, Ventile, Steuerungen und gegebenenfalls auch Batterien für einen Notstrombetrieb der elektrischen Ventilsteuerungen untergebracht sind. Das Gehäuse nimmt auch die erwähnten Atemgasanschlüsse und Anschlüsse für die Beatmungsschläuche auf. Die Anschlüsse sind im Inneren des Gerätes mit den Ventilen und mit dem Gasvorratsbehälter über Schläuche verbunden. Da sowohl diese Schläuche ein bestimmtes Bauvolumen einnehmen, als auch für die Montage dieser Schläuche ausreichend Platz vorhanden sein muss, führt dies zu einem bestimmten Bauvolumen herkömmlicher Beatmungsgeräte.

Die EP0266964 offenbart ein Beatmungsgerät zur Versorgung eines Patienten mit künstlicher Beatmung gemäss des Oberbegriffs von Anspruch 1.

### Aufgabe und Lösung

Aufgabe der Erfindung ist es, das Bauvolumen von Beatmungsgeräten zu reduzieren. Gelöst wird diese Aufgabe durch die Merkmale des Anspruches 1. Die Unteransprüche stellen bevorzugte Ausbildungen des Beatmungsgerätes das. Ein wesentliches Merkmal ist der Ersatz der Schläuche durch starre Leitungen. Ein weiteres Merkmal liegt in der Integration der Leitungen mit dem Gasvorratsbehälter zu einem kompakten Block aus Kunststoff oder Metall. In diesem Block sind folglich auch die erforderlichen Ventile und die Gasanschlüsse integriert oder an diesem angeflanscht. Das Gerät baut derart sehr kompakt.

Ausbildungen der Erfindung, die neu zum Einsatz gelangen können, umfassen insbesondere:

Das Integrieren eines Kompressors innerhalb des Gehäuses, wobei der Kompressordruckausgang gegebenenfalls über einen Schlauch mit dem Block verbunden ist, um eine schwingungsfreie Druck-Kopplung zwischen Kompressor und Block zu ermöglichen.

Beheizte Gasführungen zur Vermeidung von unerwünschter Kondensatbildung im Beatmungsgas, wobei dazu die bei der Kompressionsarbeit des Kompressors entstehende Wärme genutzt wird.

Das Erreichen einer vollständigen elektrischen Netzautarkie, indem alle elektrisch betriebenen Teile und somit auch der Kompressor von einer internen Batterie mit Strom versorgbar sind, die vorzugsweise grundsätzlich als Energiequelle dient und nur beim Vorhandensein einer Netzeinspeisemöglichkeit vom Netz über eine Ladevorrichtung laufend nachgeladen bzw. aufgeladen wird. Das Abkoppeln der Netzversorgung führt somit erfindungsgemäss zu keinerlei Betriebsunterbrechung bei der Beatmung. Auch entfällt das früher erforderlichen Anschliessen einer Druckgasversorgung mittels früher zwingend mitzutransportierender Druckgasflasche. Dies wirkt sich vorteilhaft beim Transport eines zu beatmenden Patienten aus, da das Beatmungsgerät ohne weitere Massnahmen vom Netz getrennt und mit dem Patienten mitgeführt werden kann.

Eine integrierte Ladevorrichtung für die integrierte Batterie mit einer bevorzugten Ausbildung, mit welcher beliebige Wechselstrom-Netzspannungen zwischen 80 und 270 Volt eingespeist werden können, ohne dass am Netzgerät durch Bedienpersonen Einstellungen vorgenommen werden müssen.

Einen integrierten Anschluss für die Verbindung der Elektrik bzw. Elektronik des Beatmungsgerätes mit einer externen Gleichstromquelle, z.B. mit dem Bordnetz eines Kraftfahrzeuges.

Ein in das Gehäuse integriertes Display und ein Controlpanel. Bevorzugt umfasst letzteres einen Druckdrehknopf, der es erlaubt, in Einhandbedienung Felder und Buttons am Display auszuwählen. Die Elektronik ist dabei so programmiert, dass ausgewählte Felder farblich hinterlegt erscheinen und so die Bedienung erleichtern und die Bedienungssicherheit erhöhen. Bevorzugt sind zusätzlich Tasten vorgesehen, deren Betätigungen unverzüglich Steueroperationen bzw. Programmschritte oder Einstellungen auslösen.

Elektrische Schnittstellen im Gehäuse, die einen Computeranschluss (RS 232 Schnittstelle), einen Schwesternruf, o.dgl. darstellen.

Mit dem Block verbundene oder in ihn integrierte Sensoren, die die Überwachung der Atemtätigkeit eines Patienten und die Darstellung dieser Werte auf dem Display erlauben. Eine durch die Sensoren parametrisierte Software erlaubt, wie an sich bekannt, auch die Steuerung der Beatmung in Abhängigkeit von den gemessenen Parametern.

Proximale Flusssensoren, die anschliessbar sind, um eigene Atemanstrengungen des Patienten in unmittelbarer Patientennähe zu erfassen und an die Elektronik des Gerätes liefern zu können.

Eine besondere, neue und unabhängig einsetzbare Software, die es ermöglicht, eine erzwungene Seufzerbeatmung in beliebig einstellbaren Intervallen und mit beliebig einstellbaren Druck- und/oder Voluminawerten durchzuführen-Solche Seufzerbeatmungen sind an sich bekannt, jedoch ermöglichen Geräte aus dem Stand der Technik lediglich ein unspezifisches Durchführen solcher Beatmungen. So kann bei herkömmlichen Geräten die Seufzerbeatmung aktiviert oder deaktiviert werden, d.h., dass z.B. jeder hundertste Atemzug mit 120% des normalen Atemvolumens durchgeführt wird und so die Lunge des Patienten bei jedem hundertsten Atemzug etwas überdehnt wird. Bisher ging man davon aus, dass dies ausreichend sei, da eine vergleichbare Seufzeratemfrequenz bei durchschnittlichen Patienten ermittelt wurde. Der Erfinder fand jedoch heraus, dass die durchschnittliche Seufzerbeatmung nicht immer ideal ist. So ist eine solche Beatmung z.B. bei Patienten mit einer frischen Brustkorboperation u.U. sogar schmerzhaft. Mit der erfindungsgemässen Einstellbarkeit kann nun individuell auf die Bedürfnisse jedes Patienten Rücksicht genommen werden.

### Beschreibung eines bevorzugten Ausführungsbeispieles

In den Figuren ist ein bevorzugtes Ausführungsbeispiel der Erfindung beispielhaft und nicht einschränkend für die Erfindung, dargestellt. Es zeigen dabei:
- Fig.1: ein neuartiges Beatmungsgerät im zusammengebauten Zustand auf einem fahrbaren Gestell;
- Fig.2: das Gerät nach Fig.1 beim Zusammenbau;
- Fig.3: einen erfindungsgemässen Block als zentralen Teil der Fig.2 und
- Fig.4: ein Blockschema des Ausführungsbeispieles.

Die Figuren sind übergreifend beschrieben, die angefügte Bezugszeichenliste ist Bestandteil der Beschreibung. Aus der Figurenbeschreibung ergeben sich weitere Vorteile und Merkmale der Erfindung sowie weitere bevorzugte Ausgestaltungen.

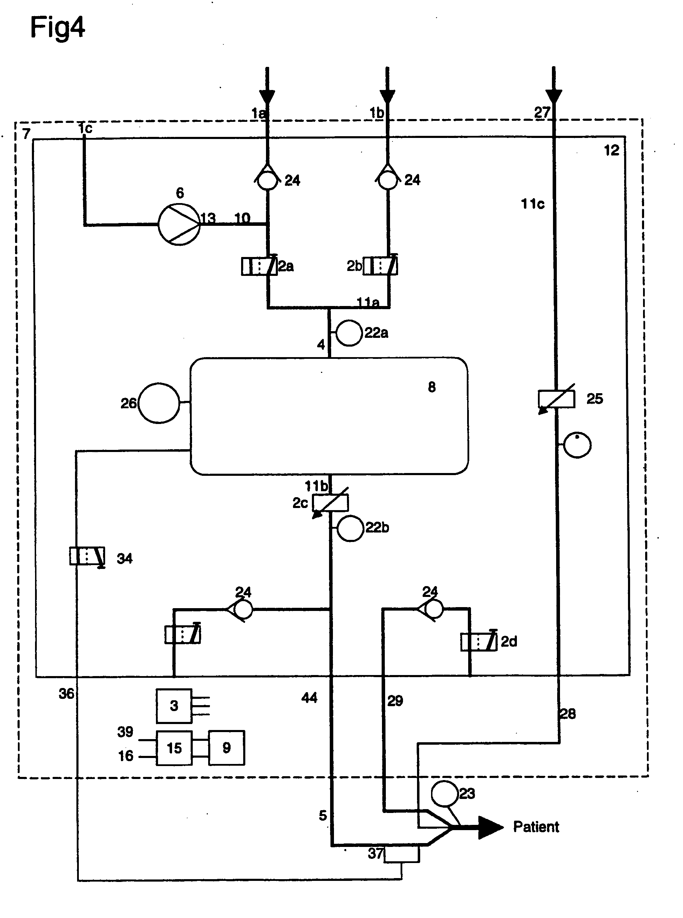

Die Funktionsweise bzw. der Aufbau des bevorzugten Ausführungsbeispieles ist aus dem Blockschema der Fig. 4 ersichtlich: eingangsseitig befinden sich Gasanschlüsse 1c als Kompressoreingang, 1a als Drucklufteingang, 1b als Atemgaseingang, z.B. als Sauerstoffeingang und 27 als Therapiegaseingang z.B. für NO. Die Eingänge sind an einem Block 12 angebracht, der innerhalb eines Gehäuses 7 untergebracht ist. 1c führt zu einem Kompressor 6, der über eine flexible Leitung 10 mit einer starren Leitung 11a verbunden ist, die eine Druckgasleitung 4 in einen Gasvorratsbehälter 8 bildet. Die Leitung 11a ist durch ein Ventil 2a unterbrochen, das die Druckluft aus dem Kompressor oder aus dem Druckluftanschluss 1a zum Gasvorratsbehälter 8 führt oder sperrt. Ein vergleichbares Atemgasventil 2b führt oder sperrt den Atemgaszugang zur Leitung 11a vom Atemgasanschluss 1b (in diesem Fall z.B. Sauerstoff).

Parallel zur Leitung 11a ist als Neuheit ebenso eine starre Leitung 11c durch den Block 12 geführt bzw. insbesondere in der Blockwandung ausgebildet, welche Leitung 11c den Therapiegasanschluss 27 mit einem Therapiegasausgang 28 verbindet. Die Leitung 11c ist durch ein regelbares Therapiegasventil 25 geteilt. Dieser Aufbau hat gegenüber bekannten Aufbauten, bei denen Therapiegasleitungen und Therapiegasregelventile separat vom Beatmungsgerät untergebracht wurden und daher zusätzlichen apparativen Aufwand bedeuteten, bevorzugt. Nicht nur der reduzierte Gehäuseaufwand und das reduzierte Bauvolumen, sondern auch die einfachere Bedienung und übersichtlichere Handhabung sind gegenüber dem Bekannten vorteilhaft.

Der Gasvorratsbehälter 8 ist mit einem Sauerstoffsensor 26 ausgerüstet. Ausgangsseitig ist er über eine starre Leitung 11b mit dem Beatmungsschlauch 5 verbunden. Die starre Leitung 11b weist ein regelbares Inspirationsventil 2c auf. Sowohl vor dem Gasvorratsbehälter 8 als auch nach dem Gasvorratsbehälter 8 wird über integrierte Sensoren 22a und 22b der Druck und/oder der Fluss gemessen. Ein proximaler Flusssensor 23 ermöglicht die Messung der Eigenatemleistung eines Patienten.

Zur Gabe von Medikamenten und Feuchtigkeit ist eine Verneblerleitung 36 vorgesehen, die ein Verneblerventil 34 und eine Verneblerkammer 37 aufweist. Verschiedene Rückschlagventile 24 verhindern Druckverlust bei allfällig abgekoppelten Anschlussschläuchen. Ein Expirationsventil 2d schliesst eine Expirationsleitung 29 ab. Ein Beatmungsschlauch 5 und bei Bedarf auch ein Therapiegasausgang 28 sind parallel zur Expirationsleitung 29 zum Patienten geführt.

Das Gehäuse 7 nimmt weiters eine Steuerung bzw. Elektronik 3 auf, die symbolisch angedeutet ist und insbesondere mit den elektronisch geregelten Ventilen und Sensoren verbunden ist. Weiters nimmt es eine Batterie bzw. einen Akkumulator 9 auf, der von einer Ladevorrichtung 15 gespeist wird. Neu und bevorzugt ist die Ladevorrichtung 15 so ausgebildet, dass sie einerseits über einen Gleichstromanschluss 16 an ein Gleichstromnetz angeschlossen und andererseits über einen Wechselstromanschluss 39 mit Wechselspannung zwischen 80 und 270V beschickt werden kann, ohne manuelle Einstellungen vornehmen zu müssen. Eine dementsprechende, an sich bekannte Elektronik ist in der Ladevorrichtung 15 integriert. Die Verbindung zwischen Batterie 9 und Ladevorrichtung 15 ist so gestaltet, dass die Batterie permanent nachgeladen wird, sofern die Ladevorrichtung 15 am Netz angeschlossen ist. Beim Ausfall der externen Stromversorgung wird automatisch auf die interne Batterie umgeschaltet.

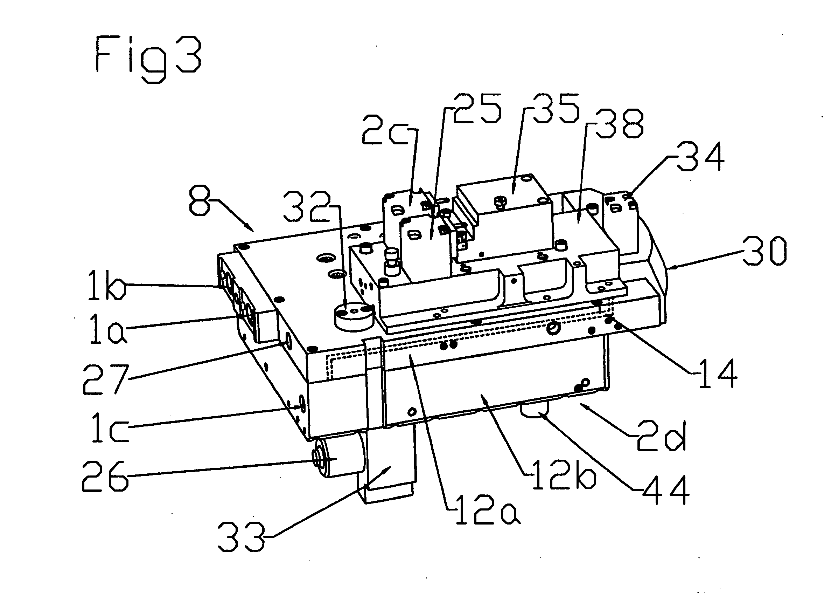

Bei diesem Ausführungsbeispiel ist der Block zweigeteilt in einen unteren Teil 12b und einen oberen Teil 12a. Dies hat Montagegründe und ist nicht zwingend. Ebenso könnten einteilige oder mehrteilige Blöcke erfindungsgemäss zur Anwendung gelangen. Wie in Fig. 3 ersichtlich, trägt unter anderem der Unterteil 12b einen Sauerstoffblock 33 mit dem Sauerstoffsensor 26 und das Expirationsventil 2d. Der Unterteil 12b ist im Betriebszustand mit dem Oberteil 12a dicht verschraubt, so dass sich im Inneren der Gasvorratsbehälter 8 ausbildet.

In den Mantelwänden des Ober- und Unterteils 12a und 12b sind erfindungsgemäss starre Leitungen ausgebildet, die die Gaswege entsprechend dem Blockschema in Fig. 4 aufnehmen. Eine besondere Leitung ist symbolisch strichliert eingezeichnet: Die Gasführung 14 ist eine Leitung, die mit dem Kompressorausgang 13 verbunden ist. Durch die Kompressionsarbeit ist bekanntlich die komprimierte ausgangsseitige Kompressorluft erwärmt. Durch das relativ lange Führen dieser erwärmten Luft in der Gasführung 14 wird diese Wärme an den Block 12 abgegeben. Dies reduziert die Gefahr einer Kondenswasserbildung im Gasvorratsbehälter 8. Alternativ zur dargestellten Gasführung 14 könnte auch im Inneren des Gasvorratsbehälters 8 eine längere, z.B. spiralförmig gewundene, Leitung den Zugang zum Kompressorausgang 13 bilden.

Der Oberteil 12a trägt unter anderem die Atemgasanschlüsse 1a und 1b sowie den Therapiegasanschluss 27. Weiters trägt er unter anderem ein Tanküberdruckventil 32, das Inspirationsventil 2c, das Therapiegasventil 25, einen Sicherheitsblock 35, der ein Patientenunterdruckventil und ein Patientenüberdruckventil aufnimmt. Die letztgenannten Ventile befinden sich auf einem ebenso als integrierten Block ausgebildeten Ventilblock 38, der insbesondere die Sicherheitsventile, die Vorrichtungen für Flussmessung und Druckmessung aufnimmt. Weiters trägt der Oberteil 12a ein Verneblerventil 34 und die Frontanschlüsse 30.

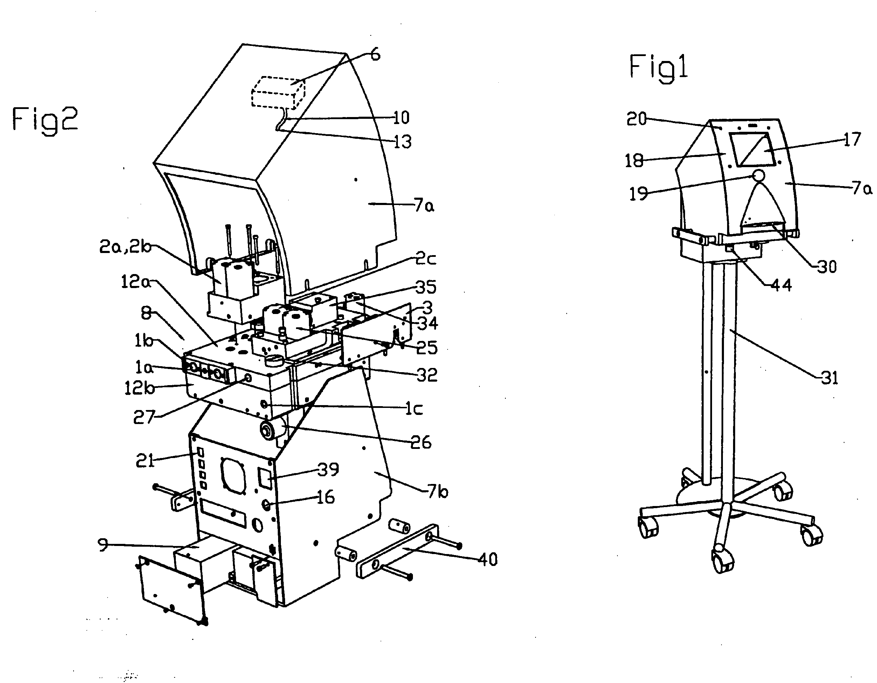

Fig. 2 zeigt im wesentlichen den Aufbau nach Fig. 3 während seines Einbaus in das Gehäuse 7, das bei dem vorliegenden Ausführungsbeispiel zweiteilig (7a, 7b) ausgebildet ist.

Symbolisch ist der Kompressor 6 mit seiner flexiblen Leitung 10 dargestellt. Im Unterteil des Gehäuses 7b erkennt man ein Fach mit eingebauter Batterie 9, Elektronik 3 und einen Platz für einen Gleichstromanschluss 16, einen Wechselstromanschluss 39 sowie eine Schnittstelle 21 für die verschiedensten Anschlüsse, wie z.B. RS232, Schwesternruf, usw.

Seitlich angebrachte Griffe 40 dienen dem Transport ebenso wie ein Gestell (Stativ) 31, das in Fig. 1 dargestellt ist.

In Fig. 1 sind weiters ein Display 17 und ein Keyboard 18 mit Druckknöpfen 20 dargestellt, sowie ein bevorzugt verwendeter Druckdrehknopf 19. Mit diesem läßt sich, wie in der Beschreibungseinleitung angegeben, besonders vorteilhaft die menügesteuerte Geräteeinstellung durchführen.

### Bezugszeichenliste

- 1: Atemgasanschluss
- 2: Ventil
- 3: Steuerung, Elektronik
- 4: Druckgasleitung, führt einerseits Druckluft und oder Kompressorluft und/oder Atemgas, das unter Druck eingespeist wird;
- 5: Beatmungsschlauch
- 6: Kompressor
- 7: Gehäuse
- 8: Gasvorratsbehälter
- 9: Batterie, Akkumulator
- 10: Schlauch im Inneren des Gehäuse
- 11: Leitung starre
- 12: Block
- 13: Kompressorausgang
- 14: Gasführung
- 15: Ladevorrichtung, ein oder mehrere Ladegeräte
- 16: Anschluss für Gleichstromnetz
- 17: Display
- 18: Controlpanel
- 19: Druckdrehknopf
- 20: Tasten
- 21: Schnittstellen
- 22: Sensor integrierter
- 23: Flusssensor proximaler
- 24: Rückschlagventil
- 25: Therapiegasventil
- 26: Sauerstoffsensor
- 27: Therapiegasanschluss
- 28: Therapiegasausgang
- 29: Expirationsleitung
- 30: Frontanschlüsse für die Leitungen zum Patienten
- 31: Gestell (Stativ)
- 32: Tanküberdruckventil
- 33: Sauerstoffblock
- 34: Verneblerventil
- 35: Sicherheitsblock
- 36: Verneblerleitung
- 37: Verneblerkammer
- 38: Ventilblock
- 39: Wechselstromanschluss
- 40: Griffe
- 44: Anschluss für Beatmungsschlauch

## Patentansprüche

1. Beatmungsgerät, unter Ausschluss von Anästhesiegeräten mit geschlossenem Atmungskreislauf, mit einem Gehäuse (7), das mit einem Gasvorratsbehälter (8) kombiniert ist, wenigstens einem Atemgasanschluss (1) und einem Kompressorluftanschluss, wenigstens einem Ventil (2) und einer elektronischen Steuerung (3) für das Ventil (2), wenigstens einem Anschluss (44) für einen Beatmungsschlauch (5) und in der Mehrzahl starren, in einem kompakten Block (12) aus Kunststoff eingebauten Gasleitungen (11) im Inneren des Gehäuses (7), in welchen Block (12) wenigstens ein Ventil (2) und wenigstens ein Anschluss (1, 44) integriert sind, **dadurch gekennzeichnet, dass** in dem Block (12) auch der Gasvorratsbehälter (8) untergebracht ist, welcher innerhalb des Blocks (12) mit den Ventilen (2) und Anschlüssen (1, 44) verbunden ist, dass innerhalb des Gehäuses (7) ein elektrisch betreibbarer Kompressor (6) angeordnet ist, dessen Kompressorausgang (13) über eine Druckgasleitung (4) mit dem Gasvorratsbehälter (8) verbunden ist, **dass** der Block (12) wenigstens eine beheizte Gasführung (14) aufweist, die mittels Kompressorabwärme beheizt ist, wobei die Gasführung (14) durch eine in der Gasvorratsbehälterwand ausgebildete Leitung von wenigstens 100 mm Länge oder von einem wenigstens 100 mm langen Rohr, das in den Gasvorratsbehälter ragt, gebildet ist, und **dass** auf seiner Gasführungsseite Mittel vorhanden sind, die wenigstens drei unterschiedliche Betriebsarten getrennt (hintereinander) oder kombiniert (gleichzeitig) erlauben: Druckluft/Anschluss an externes Druckluftnetz oder eine Druckluftflasche; Druckluft/Anschluss über integrierten Kompressor (6); Atemgas/Anschluss an externes Atemgasnetz oder an eine externe Atemgasflasche; Therapiegas/Anschluss an eine externe Therapiegasflasche.

2. Beatmungsgerät nach Anspruch 1, **dadurch gekennzeichnet, dass** auf seiner elektrischen Seite Mittel vorhanden sind, die wenigstens drei unterschiedliche befehlsfrei und unterbrechungslos wechselbare Betriebsarten getrennt (hintereinander) oder kombiniert (gleichzeitig) erlauben: Wechselspannung/Anschluss an externes Netz; Gleichspannung/Anschluss an ein Gleichstromnetz oder eine externe Batterie; Batterie/Akku/Anschluss an die interne Batterie (9).

3. Beatmungsgerät nach einem der vorhergehenden Ansprüche, **dadurch gekennzeichnet, dass** parallel zur Atemgas- bzw. Druckgasführung über wenigstens eine von der übrigen Gasführung getrennte starre Leitung (11c) eine Therapiegasführung vorhanden ist, wobei diese über dieselbe Elektronik (3) für die Atemgas- bzw. Druckgasführung geregelt ist, und wobei die starre Leitung (11c) ebenso im Block (12) integriert ist.

4. Beatmungsgerät nach Anspruch 3, **dadurch gekennzeichnet, dass** die Therapiegasführung wenigstens ein elektronisch geregeltes Therapiegasventil (25) aufweist, über das Therapiegas proportional zum Patientengasfluss durch den Beatmungsschlauch (5) dosierbar ist.

5. Beatmungsgerät nach Anspruch 4, **dadurch gekennzeichnet, dass** das Therapiegas über einen eigenen Therapiegasausgang (28) erst im Bereich des Patienten in den Patientengasfluss gemischt wird.

6. Beatmungsgerät nach einem der vorhergehenden Ansprüche, **dadurch gekennzeichnet, dass** der elektronische Steuerung (3) und dem Kompressor (6) innerhalb des Gehäuses (7) eine interne Stromversorgung, insbesondere ein Akkumulator bzw. eine Batterie (9) zugeordnet ist, die eingangsseitig mit dem Ausgang wenigstens einer integrierten Ladevorrichtung (15) verbunden ist, die eingangsseitig wenigstens zwei unterschiedliche Stromspeisearten zulässt.

7. Beatmungsgerät nach Anspruch 6, **dadurch gekennzeichnet, dass** die Ladevorrichtung eingangsseitig für den manipulationsfreien und unterbrechungsfreien Anschluss an eine Wechselspannungsquelle mit einer Spannung oder einem Spannungsbereich zwischen 80 und 270 V angeschlossen ist und/oder dass eingangsseitig ein Anschluss (16) für die Verbindung mit einem Gleichstromnetz angeordnet ist.

8. Beatmungsgerät nach einem der vorhergehenden Ansprüche, **dadurch gekennzeichnet, dass** der Block (12) wenigstens einen integrierten Sensor (22, 26) zur Gerätesteuerung aufweist und dass auf seiner elektrischen Seite Mittel vorhanden sind, die wenigstens drei unterschiedliche befehlsfrei und unterbrechungslos wechselbare Betriebsarten (Notlaufart) getrennt (hintereinander) oder kombiniert (gleichzeitig) erlauben: Wechselspannung/Anschluss an externes Netz; Gleichspannung/Anschluss an ein Gleichstromnetz oder eine externe Batterie; Batterie/Akku/Anschluss an die interne Batterie (9).

9. Beatmungsgerät nach einem der vorhergehenden Ansprüche, **dadurch gekennzeichnet, dass** die elektronische Steuerung (3) eine Software aufweist, die über ein Controlpanel (18) eine Programmierung zur Einstellung einer Seufzerbeatmung eines Patienten nach beliebig einstellbaren Zeitintervallen und nach beliebig einstellbaren Seufzervolumina und/oder nach beliebig einstellbarem Seufzerbeatmungsdruck ermöglicht.

10. Beatmungsgerät nach einem der vorhergehenden Ansprüche, **dadurch gekennzeichnet, dass** es einen Vernebleranschluss bzw. eine Verneblerleitung (36) und eine programmierbare Software für die elektronische Steuerung (3) aufweist, die über ein bzw. über das Controlpanel (18) -im Betrieb die Verneblerfunktion über die Verneblerleitung (36) und über wenigstens ein programmgesteuert geregeltes Verneblerventil (34) so steuert, dass eine Vernebelung in bestimmten Intervallen und während der Vernebelung über eine Zeitdauer und/oder eine Vernebelung in Abhängigkeit von der Einatemdynamik eines Patienten eingestellt ist.

11. Beatmungsgerät nach einem der vorhergehenden Ansprüche, **dadurch gekennzeichnet, dass** im Inneren des Gehäuses (7) eine Batterie (9) zum Betrieb des Kompressors (6) zur Gasdruckerzeugung und Gassensoren (22) untergebracht ist.

12. Beatmungsgerät nach Anspruch 11, **dadurch gekennzeichnet, dass** es Notlaufeigenschaften aufweist, indem bei einem Ausfall einer externen elektrischen und/oder pneumatischen Versorgung über den eingebauten Kompressor (6) samt Batterie (9) die Aufrechterhaltung der Beatmung sichergestellt ist.

## Claims

1. Breathing apparatus, excluding anaesthesia apparatuses with closed respiration circuit, with a housing (7), which is combined with a gas reservoir (8), at least one respiratory gas connection (1) and a compressor air connection, at least one valve (2), and an electronic control system (3) for the valve (2), at least one connection (44) for a breathing tube (5) and a plurality of rigid gas conduits (11) built into a compact block (12) of plastic in the interior of the housing (7), into which block (12) at least one valve (2) and at least one connection (1, 44) are integrated, **characterized in that** the block (12) also accommodates the gas reservoir (8), which is connected inside the block (12) to the valves (2) and connections (1, 44), **in that** the interior of the housing (7) accommodates an electrically operable compressor (6) whose compressor outlet (13) is connected to the gas reservoir (8) via a compressed gas conduit (4), **in that** the block (12) has at least one heated gas delivery means (14) which is heated by means of compressor waste heat, the gas delivery means (14) being formed by a conduit formed in the wall of the gas reservoir and having a length of at least 100 mm, or by a tube which is at least 100 mm in length and extends into the gas reservoir, and **in that**, on its gas delivery side, means are present which permit at least three different operating modes either separately (one after another) or combined (simultaneous): compressed air/connection to external compressed air network or a bottle of compressed air; compressed air/connection via integrated compressor (6); respiratory gas/connection to external respiratory gas network or to an external bottle of respiratory gas; therapy gas/connection to an external bottle of therapy gas.

2. Breathing apparatus according to Claim 1, **characterized in that,** on its electrical side, means are present which permit at least three different operating modes that are command-free and continuously interchangeable, either separately (one after another) or combined (simultaneous): alternating voltage/connection to external network; direct voltage/connection to a direct current network or an external battery; battery/accumulator/connection to the internal battery (9).

3. Breathing apparatus according to one of the preceding claims, **characterized in that,** parallel to the respiratory gas or compressed gas delivery, therapy gas is delivered via at least one rigid conduit (11c) separate from the other gas delivery, this therapy gas delivery being controlled by the same electronics (3) as for the respiratory gas or compressed gas delivery, and the rigid conduit (11c) likewise being integrated in the block (12).

4. Breathing apparatus according to Claim 3, **characterized in that** the therapy gas delivery comprises at least one electronically controlled therapy gas valve (25) via which therapy gas can be metered through the breathing tube (5) in proportion to the patient gas flow.

5. Breathing apparatus according to Claim 4, **characterized in that** the therapy gas is mixed into the patient gas flow only in the area of the patient, via a dedicated therapy gas outlet (28).

6. Breathing apparatus according to one of the preceding claims, **characterized in that** the electronic control system (3) and the compressor (6) within the housing (7) are assigned an internal power supply, in particular an accumulator or a battery (9), which on the input side is connected to the output of at least one integrated charging device (15), which on the input side permits at least two different types of power feed.

7. Breathing apparatus according to Claim 6, **characterized in that** the charging device is connected on the input side for manipulation-free and interruption-free connection to an alternating voltage source with a voltage or voltage range of between 80 and 270 V and/or **in that** an input-side connection (16) is provided for connection to a direct-current network.

8. Breathing apparatus according to one of the preceding claims, **characterized in that** the block (12) has at least one integrated sensor (22, 26) for apparatus control, and **in that**, on its electrical side, means are present which permit at least three different operating modes that are command-free and continuously interchangeable (emergency mode), either separately (one after another) or combined (simultaneous): alternating voltage/connection to external network; direct voltage/connection to a direct-current network or an external battery; battery/accumulator/connection to the internal battery (9).

9. Breathing apparatus according to one of the preceding claims, **characterized in that** the electronic control system (3) comprises software which, via a control panel (18), permits programming in order to set sigh ventilation for a patient at adjustable time intervals and at adjustable sigh volumes and/or at an adjustable sigh ventilation pressure.

10. Breathing apparatus according to one of the preceding claims, **characterized in that** it has a nebulizer connection or a nebulizer conduit (36) and programmable software for the electronic control system (3) which, via a control panel or the control panel (18), controls the nebulizer function via the nebulizer conduit (36) and via at least one program-controlled nebulizer valve (34) during operation in such a way that nebulization is set at certain intervals and during nebulization over a time period and/or nebulization dependent on the inhalation dynamics of a patient.

11. Breathing apparatus according to one of the preceding claims, **characterized in that** the interior of the housing (7) accommodates a battery (9) for operation of the compressor (6) for gas pressure generation and gas sensors (22).

12. Breathing apparatus according to Claim 11, **characterized in that** it has emergency operation features which, in the event of a failure of an external electrical and/or pneumatic supply, ensure that ventilation is maintained via the integrated compressor (6) and battery (9).

## Revendications

1. Appareil respiratoire, à l'exception des appareils d'anesthésie avec circuit respiratoire fermé, comprenant un boîtier (7) qui est combiné avec un réservoir à gaz (8), au moins un raccord à gaz respiratoire (1) et un raccord à air comprimé, au moins une valve (2) et une commande électronique (3) pour la valve (2), au moins un raccord (44) pour un tuyau respiratoire (5) et une pluralité de conduites de gaz (11), pour la plupart rigides, montées dans un bloc compact (12) en matière plastique à l'intérieur du boîtier (7), bloc (12) dans lequel sont intégrés au moins une valve (2) et au moins un raccord (1, 44), **caractérisé en ce que** dans le bloc (12) est également logé le réservoir à gaz (8), lequel est relié avec les valves (2) et les raccords (1, 44) à l'intérieur du bloc (12), qu'à l'intérieur du boîtier (7) est disposé un compresseur (6) à commande électrique dont la sortie de compresseur (13) est reliée avec le réservoir à gaz (8) par le biais d'une conduite de gaz sous pression (4), que le bloc (12) présente au moins un dispositif d'acheminement de gaz (14) chauffé qui est chauffé au moyen de la chaleur dissipée par le compresseur, le dispositif d'acheminement de gaz (14) étant formé par une conduite d'au moins 100 mm de long façonnée dans la paroi du réservoir à gaz ou par un tube d'au moins 100 mm de long qui fait saillie dans le réservoir à gaz et que sur son côté acheminement du gaz existent des moyens qui permettent au moins trois modes de fonctionnement différents séparément (l'un après l'autre) ou combinés (simultanés) : air comprimé/raccordement à un réseau d'air comprimé ou à une bouteille d'air comprimée externe ; air comprimé/raccordement par le biais du compresseur intégré (6) ; gaz respiratoire/ raccordement à un réseau de gaz respiratoire ou à une bouteille de gaz respiratoire externe ; gaz thérapeutique/raccordement à une bouteille de gaz thérapeutique externe.

2. Appareil respiratoire selon la revendication 1, **caractérisé en ce que** des moyens sont présents sur son côté électrique, lesquels permettent au moins trois modes de fonctionnement différents sans instruction et commutables sans interruption séparément (l'un après l'autre) ou combinés (simultanés) : tension alternative/raccordement à un réseau externe ; tension continue/raccordement à un réseau électrique continu ou à une batterie externe ; pile/accu/ raccordement à la batterie interne (9).

3. Appareil respiratoire selon l'une des revendications précédentes, **caractérisé en ce qu'**il existe un dispositif d'acheminement de gaz thérapeutique parallèle au dispositif d'acheminement de gaz respiratoire ou de gaz sous pression par le biais d'au moins une conduite rigide (11c) séparée du reste du dispositif d'acheminement de gaz, celui-ci étant régulé par la même électronique (3) que le dispositif d'acheminement de gaz respiratoire ou de gaz sous pression et la conduite rigide (11c) étant elle aussi intégrée dans le bloc (12).

4. Appareil respiratoire selon la revendication 3, **caractérisé en ce que** le dispositif d'acheminement de gaz thérapeutique présente au moins une valve à gaz thérapeutique (25) à régulation électronique par le biais de laquelle le gaz thérapeutique peut être dosé proportionnellement au flux de gaz patient à travers le tuyau respiratoire (5).

5. Appareil respiratoire selon la revendication 4, **caractérisé en ce que** le gaz thérapeutique est mélangé dans le flux de gaz patient par le biais d'une sortie de gaz thérapeutique (28) propre seulement à proximité du patient.

6. Appareil respiratoire selon l'une des revendications précédentes, **caractérisé en ce qu'**une alimentation électrique interne, notamment un accumulateur ou une pile (9), est associée à la commande électronique (3) et au compresseur (6) à l'intérieur du boîtier (7), laquelle est reliée du côté de l'entrée avec la sortie d'au moins un dispositif de charge (15) intégré qui autorise du côté de l'entrée au moins deux modes d'alimentation électrique différents.

7. Appareil respiratoire selon la revendication 6, **caractérisé en ce que** le dispositif de charge, pour un branchement sans manipulation et ininterrompu, est raccordée du côté de l'entrée à une source de tension alternative ayant une tension ou une plage de tension entre 80 et 270 V et/ou qu'une borne (16) pour la connexion à un réseau électrique continu est disposée du côté de l'entrée.

8. Appareil respiratoire selon l'une des revendications précédentes, **caractérisé en ce que** le bloc (12) présente au moins un capteur intégré (22, 26) pour commander l'appareil et qu'il existe de son côté électrique des moyens qui permettent au moins trois modes de fonctionnement (mode secours) différents sans instruction et commutables sans interruption séparément (l'un après l'autre) ou combinés (simultanés) : tension alternative/raccordement à un réseau externe ; tension continue/raccordement à un réseau électrique continu ou à une batterie externe ; pile/accu/raccordement à la batterie interne (9).

9. Appareil respiratoire selon l'une des revendications précédentes, **caractérisé en ce que** la commande électronique (3) présente un logiciel qui permet, par le biais d'un panneau de commande (18) une programmation destinée à régler un soupir de ventilation d'un patient d'après des intervalles de temps réglables à volonté et d'après un volume de soupir réglable à volonté et/ou d'après une pression de soupir de ventilation réglable à volonté.

10. Appareil respiratoire selon l'une des revendications précédentes, **caractérisé en ce qu'**il présente un raccord de nébuliseur ou une conduite de nébuliseur (36) et un logiciel programmable pour la commande électronique (3), lequel commande par le biais d'un ou par le biais du panneau de commande (18), en fonctionnement, la fonction de nébuliseur par le biais de la conduite de nébuliseur (36) et par le biais d'au moins une valve de nébuliseur (34) régulée commandée par programme de telle sorte qu'une nébulisation soit réglée à des intervalles donnés et, pendant la nébulisation, sur une durée donnée et/ou en fonction de la dynamique d'inspiration d'un patient.

11. Appareil respiratoire selon l'une des revendications précédentes, **caractérisé en ce qu'**à l'intérieur du boîtier (7) est logée une pile (9) pour faire fonctionner le compresseur (6) destiné à générer la pression du gaz et les capteurs de gaz (22).

12. Appareil respiratoire selon la revendication 11, **caractérisé en ce qu'**il présente des caractéristiques de fonctionnement de secours **en ce qu'**en cas de panne d'une alimentation électrique et/ou pneumatique externe, le maintien de la ventilation est garanti par le biais du compresseur intégré (6) et de la pile (9).
